# EUROPEAN PATENT SPECIFICATION

(11) **EP 1 118 616 B1**
(45) Date of publication and mention of the grant of the patent: **24.03.2004**
(21) Application number: 01100018.9
(22) Date of filing: 04.01.2001
(51) Int. Cl.: C07D 519/04

(54) **Process for the production of 5'-nor-anhydrovinblastine ditartrate from plants of genus catharanthus**
Verfahren zur Herstellung von 5'-Nor-anhydrovinblastinditartrat aus Pflanzen der Gattung Catharanthus
Procédé pour la préparation de ditartrate de 5'-nor-anhydrovinblastine à partir de plantes du genre Catharanthus

(30) Priority: 12.01.2000 AR 0000126
(43) Date of publication of application: 25.07.2001
(73) Proprietor: Eriochem, S.A., Parana, Entre Rios (AR)
(72) Inventor: Moyano, Nora, Parana, Entre Rios (AR); Iturraspe, José, Parana, Entre Rios (AR); Nunez, José Lucio, Parana, Entre Rios (AR)
(74) Representative: SUGRANES - VERDONCES - FERREGÜELA

(56) References cited:
- WO-A-88/02002
- FR-A- 2 544 318
- US-A- 4 307 100

## Description

This invention relates to a new complete procedure for industrial-scale production of 5' -nor-anhydrovinblastine ditartrate from fresh Catharanthus leaves and shoots. It involves extraction of alkaloids from the aforementioned plant, from which a mixture of Vindoline and Catharanthine is purified. This mixture is caused to react by a ferric cation as catalyser to obtain anhydrovinblastine, which is used to synthesise 5' -nor-anhydrovinblastine. The latter is used to prepare 5' -nor-anhydrovinblastine ditartrate, a bis-indolic alkaloid known to have antitumoral effects, used in chemotherapy of lung cancer, breast cancer, and Hodgkin disease.

### PRIOR ART

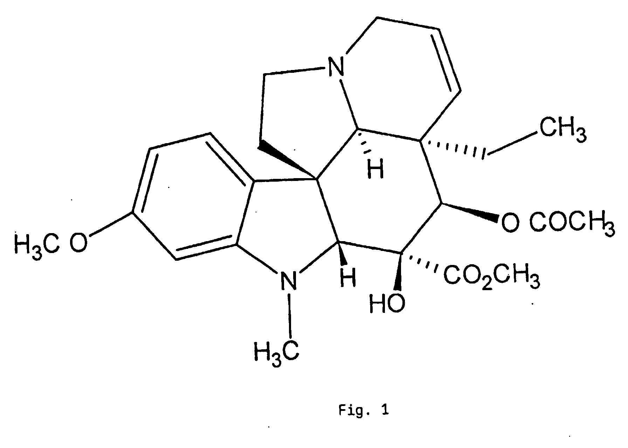

Vindoline (3-methoxycarbonyl-4-acetyloxy-6,7-didehydro-3-hydroxy-16-methoxy-1-methyl-aspidospermidine, PM: 456,54), is a dihydroindolic alkaloid. Its formula is shown in Figure 1.

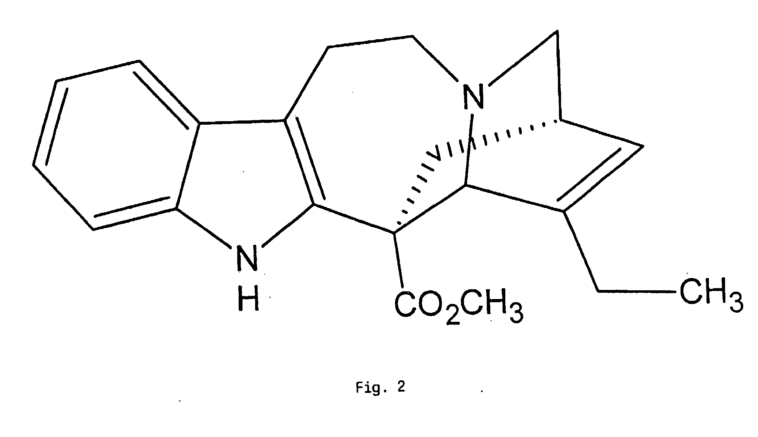

Catharanthine (18-methoxycarbonyl-3,4-didehydro-ibogamine, PM: 336,43) is an indolic alkaloid. Its formula is shown in Figure 2.

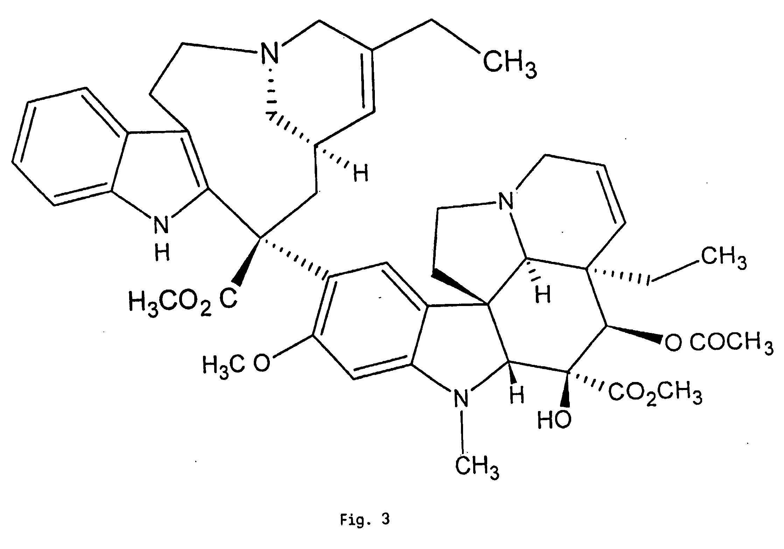

Anhydrovinblastine (3',4'-didehydro-4'-deoxy-vinblastine, PM:764,92) is a bis-indolic alkaloid. Its formula is shown in Figure 3.

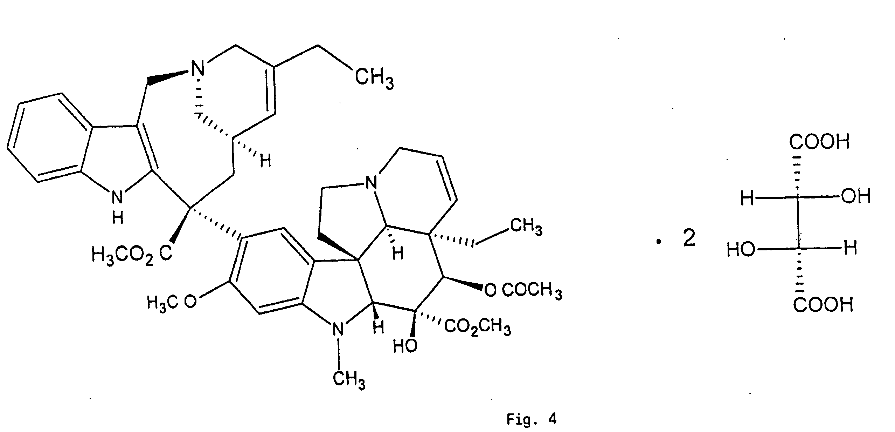

5'-nor-anhydrovinblastine ditartrate (3',4'-didehydro-4'-deoxy-C5'-nor-vinblastine, ditartrate, PM: 1079,13) is a bis-indolic alkaloid. Its formula is shown in Figure 4.

5' -nor-anhydrovinblastine is similar in structure to vinblastine. Its difference is one less methylene in the catharanthine half, and the presence of a double bond between carbons 3' and 4' due to the absence of a water molecule. 5' -nor-anhydrovinblastine bears a particular interest for its antitumoral effects and its reduced neurotoxicity if compared to vinblastine. 5' -nor-anhydrovinblastine ditartrate is a salt used for the clinical administration of the drug.

Vindoline and Catharanthine bear pharmacological interest since they are the raw material for the semi-synthesis of anhydrovinblastine, which is used as raw material for the synthesis of antitumoral bis-indolic alkaloids such as vinblastine and 5' -nor-anhydrovinblastine. Also, vinblastine may be used to synthesise vincristine and vindesine.

The natural dimeric alkaloids extracted from Vinca -vinblastine and vincristine- have effective clinical antimitotic effects due to their cell cycle blockage mechanism in phase M. They are administered for the treatment of Hodgkin disease and ovary cancer (Lhomme, C., Rev. Prat. (France) 40, 1780-1781 (1990)). In spite of their effectiveness some drawbacks in their use have been detected, such as cross resistance (Maral, R., Bounut, C., Chenn, E. and Mathe, G., Cancer Chemother. Pharmacol. 5 (3), 197-199 (1981)), and high levels of toxicity, particularly neurotoxicity (Binet, S., Chaineau, E., Fellous, A., Lataste, H., Krikorian, A., Couzinier, J.P., Meininger, V., Int. J. Cancer 46, 262-266 (1990)).

These side effects have stimulated research and development aimed at the synthesis of these alkaloid analogues, in order to maintain their antimitotic effects but lower their levels of toxicity.

A great number of semi-synthetic by-products have been chemically and pharmacologically studied and only some have reached the clinical study stage [O. Van Tellingen, J. H. M. Sips, J. H. Beijnen, A. Bult and W. J. Nooijen, Anticancer Research, 12, 1699-1716 (1992)].

5' -nor-anhydrovinblastine is the most important of the vinblastine analogues. This compound bears great interest since it has shown high levels of antitumoral activity and low levels of neurotoxicity with less side effects than vinblastine and vincristine. Its effect is based on a strong inhibiting power of tubulin polymerisation resulting in inhibition of the formation of the achromatic spindle which allows the migration of chromosomes during the mitosis anaphase (Fellous, A., Ohayon, R., Vacassin, T., Binet, S., Lataste, H., Krikorian, A., Couzinier, J.P., and Meininger, V., Semin. Oncol. 16, 9-14 (1989)).

The climate and soil conditions of Entre Rios province have proved proper for an adequate development in the cultivation of Apocinaceous plants, in particular of the Catharanthus genus, and especially the Catharanthus roseus, Catharanthus ovalis, and Catharanthus longifolius species, more specifically Catharanthus roseus G. Don, formerly known as Vinca rosea Linn. ("Index Kewensis" Part IV, Claredon Press, Oxford, England, 1895, pag.1203).

Moreover, the agricultural practise in Argentina, combined with the development of pharmaceutical technology, prioritise the development of products which, extracted from locally produced vegetable raw materials, add value to the national economy. The Research and Development. Department of Eriochem S.A. has created a new procedure -the present invention- after intense research and testing in its industrial plant. The mentioned procedure allows the synthesis of 5' -nor-anhydrovinblastine ditartrate from raw materials grown in the province of Entre Rios at an industrial-scale. Its purity is over 99%, and it is fit for use in medicinal formulae for human use.
The state of the art is rich in bibliography on partial processes such as extraction of Vindoline and Catharanthine in patents FR2544318, US4172077 among others, which obtain said isolated and purified monomeric alkaloids. Other quotes such as Pierre Potier's studies (*Synthesis of Catharanthus Alkaloids,* "Journal of Natural Products" V43,N1,jan-feb 1980), patents US4279817, US4307100, US5047528, FR2544319 and their related articles describe the synthesis of intermediate anhydrovinblastine from pure Vindoline and Catharanthine or mixtures through the formation of Catharanthine N-oxide followed by the modified Polonovsky reaction. Another synthesis method developed by Mitsui Petrochemical Ind. Ltd. uses pure Vindoline and Catharanthine as starting materials and anhydrovinblastine is obtained through oxidation with ferric cation (WO88/02002, EP354778).

Patent US4307100 claims the 5' -nor-anhydrovinblastine molecule with N-oxide as the utilised process. Other patents such as US5220016 obtain 5' -nor-anhydrovinblastine from an indol, synthesising each functional group, step by step, until the total synthesis of the molecule is achieved.

There is no information in the present state of the art regarding a process which takes Catharanthus species as starting material and obtains 5' -nor-anhydrovinblastine ditartrate. Moreover, if the scientific and patent related bibliography are juxtaposed, processes ranging from the extraction of the already ground plant to the production of crude 5'-nor-anhydrovinblastine are found, but these do not suggest the paths followed in the synthesis proposed in this patent.

Facing the technological challenge of attaining a synthesis of 5' -nor-anhydrovinblastine ditartrate from alkaloids extracted from the Catharanthus roseus species, a procedure with the following new characteristics has been developed:

The fresh plant is processed so that the desired alkaloids undergo the least possible degradation, an impure mix of Vindoline and Catharanthine is used for the synthesis of anhydrovinblastine with the ferric ion catalyser, a new method for anhydrovinblastine storage is developed, purification and recycling of solvents are performed, crude 5' -nor-anhydrovinblastine is synthesised in tandem without isolation of the bromated intermediate, crude 5' -nor-anhydrovinblastine is purified by chromatography and crystallisation, and finally a method to obtain 5' -nor-anhydrovinblastine ditartrate which does not generate loss of active matter in order to obtain the desired purity is developed. This results in a greater global yield, reduced process time, reduced use of solvents and a smaller number of purifications because it includes several reactions in single process steps. Thus, the new production process is more economical and efficient.

### DESCRIPTION

The procedure in the present invention may be divided in seven main stages, which are listed below:
1) grinding of the non-degraded Catharanthus plant
2) procurement of the total alkaloids of Catharanthus plant
3) procurement of the chromatographic fraction of Vindoline and Catharanthine
4) synthesis and purification of anhydrovinblastine
5) synthesis of 5' -nor-anhydrovinblastine
6) purification of crude 5' -nor-anhydrovinblastine
7) preparation of 5' -nor-anhydrovinblastine ditartrate

More specifically, the procedure in the present invention comprises the following process steps:
- grinding of plants of Apocinaceous family by means of a blade or hammer mill;
- solid-liquid extraction of alkaloids from the ground plant by means of an organic solvent soluble in water;
- concentration of the extract at room temperature (from 20°C to 30°C) to eliminate the organic solvent and avoid decomposition of Vindoline and Catharanthine;
- recovery of the solvent by rectification;
- dilution of the extract in an aqueous acidic solution;
- elimination of impurities and pigments of the plant extract by means of liquid-liquid extraction of the aqueous acidic dilution, with an organic solvent;
- recovery of solvent by distillation;
- neutralisation and/or alkalisation of the aqueous acidic phase with either an inorganic or organic base;
- liquid-liquid extraction with an organic solvent, which extracts the alkaloids dissolved in water,
- concentration by evaporation, at low temperature, of the total alkaloids extract;
- recovery of solvents by condensation;
- separation by liquid chromatography of the total alkaloids extract on silicagel with mixtures of organic solvents;
- isolation of the impure chromatographic fraction rich in Vindoline and Catharanthine;
- concentration until dry by evaporation at reduced pressure;
- recovery of solvents by condensation;
- synthesis of anhydrovinblastine from Vindoline and Catharanthine by dissolving them in a tamponed aqueous acidic solution and adding a ferric salt, which catalyses the binding of Vindoline to Catharanthine resulting in an oxidised iminio intermediate compound;
- tandem reduction of the oxidised iminio intermediate to anhydrovinblastine, with sodium borohydride in an ammonium hydroxide solution;
- extraction of anhydrovinblastine with an organic solvent;
- concentration by evaporation of the organic solvent at low temperature;
- recovery of solvents by condensation;
- crystallisation of anhydrovinblastine from methanol;
- filtering and vacuum drying of anhydrovinblastine;
- reaction of anhydrovinblastine and a halogenated agent at low temperature from -40° C to -80° C with an organic solvent, organic acid and nitrogen bubbling, attaining a halogenated intermediate of anhydrovinblastine;
- reaction of this intermediate with silver tetrafluoroborate in a medium alkalinised with ammonium acetate in water and tetrahydrofurane to attain crude 5' -nor-anhydrovinblastine;
- extraction with non-polar organic solvent and concentration by evaporation;
- recovery of solvents by condensation;
- purification of 5' -nor-anhydrovinblastine by chromatography and crystallisation until a concentration over 99 % is reached;
- recovery of the chromatographic eluent solvents by condensation of the evaporated fractions;
- formation of 5' -not-anhydrovinblastine ditartrate with tartaric acid in alcohol or aqueous medium;

The procedure is characterised in that, the plants of Apocinaceous family are ground fresh and moist or dried at a temperature less than 30°C; the synthesis of anhydrovinblastine is performed with an impure fraction of a mixture rich in Vindoline and Catharanthine at pH 2 for a period of between 15 to 25 hours and no Vinblastine is produced; the anhydrovinblastine is stored after its synthesis in nitrogen and at less than -20°C until its use; the reaction of said halogenated intemediate with silver tetrafluoroborate is a tandem reaction; and in that the formation of 5' -nor-anhydrovinblastine ditartrate consists of dissolution of crude 5' -nor-anhydrovinblastine, with a purity of over 99% in absolute ethanol in a concentration of 0,10 to 0,20 mmol/ml and addition of the equimolar quantity of tartaric acid and concentration until dry at reduced pressure and in the dark, or lyophilization.

The detailed steps of the new process are described below, according to the preferred procedure:

The starting material is a plant from the Apocynaceae family, in particular of the genus Catharanthus, and especially the Catharanthus roseus , Catharanthus ovalis and Catharanthus longifolius species, more particularly Catharanthus roseus G. Don, formerty known as Vinca rosea Linn, which is rich in alkaloids. Vindoline and Catharanthine are of particular interest for the present process. Laboratory tests showing the definition of the parts of the plant which contain Vindoline and Catharanthine have been performed. These tests have determined that the substances are found in significant quantities only in its leaves and shoots, throughout the year regardless of the plant's blooming period. In stems and roots, Vindoline and Catharanthine may be found in minor quantities which do not justify extraction.

The quoted bibliography mentions the traditional drying of the plant prior to the extraction of Vindoline and Catharanthine. After various laboratory tests it was concluded that both heat and time affect the concentration of the alkaloids of interest in Vinca rosea. Thus, two new alternative procedures were developed to solve the problem. The first alternative is to use the fresh, moist starting material. The grinding of fresh plants entails difficulties which have been overcome by the use of a blade mill, thus attaining a highly disintegrated material fit for extractions with solvent. The second alternative is drying the freshly harvested material at low temperature, below 35° C, and preferably below 20° C from 1 to 15 days, preferably less than 7 days. This process does not affect the concentration of alkaloids present in the fresh plant, especially Catharanthine. Thus, it is possible to use a hammer or blade mill.

A total extract of Catharanthus is obtained by means of an extraction with solvents miscible with water. This method shows advantages as compared to extraction with immiscible solvents, since a pre-treatment with alkali to liberate the alkaloids present as salts in the plant is not required, as these are soluble in alcohol. The extraction is performed at room temperature (from 20° C to 30° C) three times with aliphatic alcohols from 1 to 5 C or its mixtures. Methanol, ethanol and isopropanol may be used (Methanol is preferable), for a period of 30 minutes to 40 hours each time. The extracts are concentrated in a rising film tubular evaporator at reduced pressure, at a temperature below 30° C. The solvents are recovered by condensation, and a rectification is performed to recover the alcohol, separating it from water and impurities. After running the corresponding tests it returns to the same process step.

An inorganic acid, which may be sulphuric acid 1 N, hydrochloric acid 1 N, is added to the concentrate -which is mainly formed by water from the plant itself- and is extracted with a non-polar organic solvent, which may be methylene chlorine, chloroform, dichloroethane, hexane, petroleum ether (40-60°), isopropylic ether, ethyl acetate, preferably methylene chloride. Three consecutive extractions are performed in order to eliminate the chlorophyll and other pigments. The organic phase with these pigments is distilled and the solvent is recovered and returned to the process after analysis. The aqueous phase is alkalised with concentrated ammonium hydroxide or sodium hydroxide 1 N, preferably concentrated ammonium hydroxide, with a pH between 7.5 and 9. It is saturated with inorganic salt which could be sodium chloride, magnesium sulphate, sodium sulphate, preferably sodium chloride.

The extraction is performed with a non-polar organic solvent, such as methylene chloride, chloroform, dichloroethane, hexane, petroleum ether (40-60°), isopropylic ether, ethyl acetate, preferably methylene chloride. After three extractions, the resulting extracts are concentrated at reduced pressure in a rotary evaporator until dry, with a bath temperature of 40° C or lower. The solvents are condensed and reused in the same process step.

The result is a crude of total alkaloids with a mass yield of 0.05 to 0.3% in relation to the fresh material -typically 0.15 to 0.25%. The crude is chromatographed in a column of silicagel 60 from 63 to 37 microns (mm)(230-400 mesh ASTM), eluting with a mixture of methylene chloride-ethyl acetate 80:20. The different fractions are collected and the ones which are the richest in the mixture of Vindoline and Catharanthine are selected (the fractions are controlled by chromatography on a thin layer using Merck silicagel sheets and methylene chloride-acetone-methanol as development solvent: 92:5:3). The separated fractions are evaporated at reduced pressure in a rotary evaporator, thus attaining a mixture rich in Vindoline and Catharanthine containing 40% to 80% of the Vindoline and Catharanthine mixture in molar ratio 1:1 to 1.5:1, typically 1.1:1 to 1.3:1 as extracted from the plant. This means a mass yield of 5% to 20% of the total alkaloids, particularly between 7% and 12%. The chromatographic elution solvents are rectified, and analysed, the necessary concentration ratio is adjusted and the solvents are reused in the chromatography process.

After that, the anhydrovinblastine is synthesised with the impure mixture of Vindoline and Catharanthine with the ferric cation as catalyser. Working with an impure fraction avoids the costly separation and purification of each of these two alkaloids, thus reducing the amount of solvents and silicagel required. Reaction yields of 70% are achieved, which matches the ones mentioned in the quoted bibliography, and the ones obtained in Eriochem S.A. labs with purified Vindoline and Catharanthine as starting materials. Once the reaction to obtain anhydrovinblastine is finished, this dimeric alkaloid is isolated and purified in the same fashion, regardless of whether the purified monomers were used as starting materials or if the starting material was a mixture with desired alkaloid levels ranging from 40% to 100%. This step of the new procedure is neither suggested nor evidenced in the present state of the art.

The anhydrovinblastine synthesis is conducted in a glass reactor, in an aqueous medium. Anhydrovinblastine is better obtained at pH 2 or lower, since the higher the pH, the higher the production of Vinblastine. The medium can be formed by using a mineral acid in concentrations below 1%, to get the proper pH. Hydrochloric or sulphuric acid may be used. A tampon such as glycine or citrate, preferably a buffer of glycine, sodium chloride and hydrochloric acid, at pH 2 should be used.

The reaction is performed at a temperature between 10° C and 25° C, the mixture is bubbled in nitrogen or another inert gas, such as argon, and this condition is maintained during the reaction to avoid the formation of undesired alkaloids such as vinblastine, catharine, leurosine, etc., by oxidation.

The ferric catalyser is added to this buffer. The catalyser may be ferric chloride, ferric sulphate, ferric nitrate, preferably ferric chloride hexahydrate.

The catalyser is added in a molar ratio of 5:1 to Catharanthine.

The chromatographic fraction rich in Vindoline and Catharanthine is added while agitation and nitrogen bubbling are maintained for a period of 2 to 40 hours, during which an iminio intermediate is formed. This intermediate becomes anhydrovinblastine by reducing the reaction product with an agent, particularly sodium borohydride, in an alkaline medium, preferably concentrated ammonium hydroxide, at room temperature and letting it react 10 to 60 minutes, preferably between 10 and 15 minutes.

In order to isolate anhydrovinblastine, the mixture is extracted with non-polar organic solvent, which may be ethyl acetate, ethyl ether, preferably methylene chloride. After four extractions, the organic phase is filtered through a filter layer such as Celite in order to isolate the iron and it is concentrated at reduced pressure. The residue is diluted in methylene chloride or an organic solvent such as ethyl acetate, ethyl ether, preferably methylene chloride, and it is treated with activated carbon.
The decoloured solution is concentrated at reduced pressure. The solvent is condensed and reused in the process after analysis and the evaporation residue is dissolved in an aliphatic alcohol of less than 4 C, preferably methanol or ethanol, methanol is preferred, at room temperature and is kept at 4 to 8° C for 7 to 18 hours. The precipitate is filtered, washed in cold methanol and vacuum dried.

The yields for anhydrovinblastine are 50% to 85%, typically 65% to 75%, depending on the aforementioned conditions used.

There are references in the present state of the art on instability and the need for an immediate use of anhydrovinblastine. A method which is part of this new process has been successfully tested. It allows the storage of this alkaloid in perfect conditions for over three months. This step is performed in a nitrogen atmosphere at a temperature of -15° C to -80° C, preferably at -20 °C. This enables the setting of a production schedule which reduces the global costs due to the optimal usage of the plant equipment.

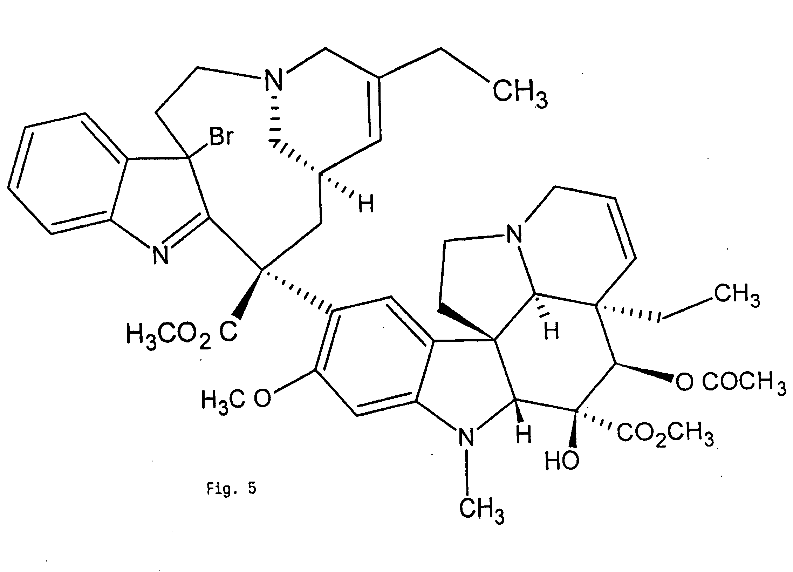

Anhydrovinblastine is reacted with a halogenation agent to produce a halogenated intermediate in position 7'. Several halogenation agents may be used, such as chlorobenzotriazol, N-chlorosuccinimide or N-chloroacetamide, preferably N-bromosuccinimide, which specifically produces intermediate 7'-bromoindolenine from anhydrovinblastine, as shown in Figure 5.

The stechiometric proportions of the reactants could be between 0.8:1 and 1.3:1, preferably between 1:1 and 1.15:1 (halogenating agent for anhydrovinblastine), and the reaction is favoured by the presence of 1 to 2 equivalents of trifluoroacetic acid.

The reaction is performed in an inert atmosphere (nitrogen or argon), at low temperatures, -80° C to -40° C, preferably -50° C to -65° C. These conditions should be maintained during the whole reaction by adding all the reactives, for a period of 1 to 4 hours, preferably 2 hours.

In the present state of the art, both in the work of Françoise Gueritte, *Composés antitumoraux du grupe de la vinblastine: dérivés de la nor-5' anhydrovinblastine* (Eur. J. Med. Chem. Chim. Ther. 1983-18 n5 pp 419-424), and in the US patent 4.307.100, the obtained halogenated derivative is isolated by extraction with solvents after neutralisation of the reaction medium with sodium hydroxide or a similar base.

In this new procedure, the transformation of the bromated intermediate into 5'-nor-anhydrovinblastine is performed in tandem with the bromation by addition to the same reaction medium, after neutralisation with ammonium acetate, silver tetrafluoroborate in an aqueous medium, or mixtures of water and an organic solvent such as tetrahydrofurane. After adding silver tetrafluoroborate, the mixture is left to react at room temperature for 2 to 18 hours, preferably for 3 to 5 hours and is then alkalinised with sodium bicarbonate or sodium acetate. The product is extracted from the aqueous phase with non-polar organic solvent, preferably methylene chloride. Through this procedure, the isolation of 7'-bromoindolenine from anhydrovinblastine -an intermediate which is rather unstable- is avoided. This along with the resulting yield increase, make the production of this compound at an industrial scale economically feasible.

The added extracts are dried up on anhydrous sodium sulphate, filtered and the solvent is evaporated at reduced pressure, condensed and reused. The reaction yield is between 10 and 60%, typically between 25 and 40%.

Next, the crude 5' -nor-anhydrovinblastine is purified by flash column chromatography using silica gel as support, granulometry from 63 to 37 microns (mm) (230-400 mesh ASTM) and eluting with mixtures of acetone and methanol in ratios ranging from 100:0 to 80:20, preferably in azeotropic relationship (88:12 approximately), which allows the mixture recovery simply by condensation after concentration of the 5' -nor-anhydrovinblastine.

The composition of the fractions is monitored by thin layer chromatography (Merck silicagel sheets, development solvent methylene chloride-hexane-methanol 50:40:10, detection at 254nm).

After the flash column chromatography the solvent is evaporated and a product rich in 5' -nor-anhydrovinblastine is obtained. For the fractions which contain additional components, a second chromatography under the same conditions is required.

The fractions with 5' -nor-anhydrovinblastine as the only stains in the thin layer chromatography, are concentrated at reduced pressure and the residue is recrystallised with methanol, dissolving it at a temperature between 10 and 60° C, more particularly between 20° C and 30° C, allowing it to crystallise at a low temperature, between 5° C and -30° C, preferably between 2° C and -20° C. It is then filtrated and dried up. The material purity is determined by HPLC, and it is over 99%.

This material is 5' -nor-anhydrovinblastine in its crude form. The form used to obtain the final pharmaceutical product is 5' -nor-anhydrovinblastine ditartrate.

It is known that the usual method of purifying salts such as alkaloids tartrates is by means of a dissolution in anhydrous alcohol and crystallisation, with addition of hexane or ethyl ether to contribute to precipitation. In Eriochem S. A.'s labs we have found that 5' -nor-anhydrovinblastine ditartrate in particular does not show a crystalline form, but precipitates as an amorphous material, so that the usual procedure leads to loss of active matter in the mother liquid without substantial improvement in the quality of the material.

After experimentation with different ways of forming and purifying the ditartrate the present procedure was achieved. It consists of dissolution of crude 5' -nor-anhydrovinblastine, -with a purity of over 99% (HPLC)- in absolute ethanol, in a concentration of 0.10 to 0.20 mmol/ml, preferably 0.15 to 0,19 mmol/ml and addition of the equimolar quantity of tartaric acid at room temperature, between 10° C and 50° C, preferably between 20° C and 30° C. Then, the mixture is concentrated until dry at reduced pressure. The amorphous white solid is then vacuum dried at 666 Pa(5 mmHg) or lower, between 20° C and 50° C in the dark until constant weight is reached. 5'-nor-anhydrovinblastine ditartrate is quantitatively obtained.

Alternatively, the ditartrate can be prepared in an aqueous medium, by lyophilization of a solution with concentration between 2% and 10% of 5'-nor-anhydrovinblastine ditartrate, made by dissolution of the necessary quantity of tartaric acid and addition of the corresponding crude 5' -nor-anhydrovinblastine, between 10° C and 30° C.

The mentioned material is fit for the preparation of a final pharmaceutical formula. The material purity, determined by HPLC, is over 99 % and its titer determined by titration of total alkaloids by non-aqueous volumetry and corrected for weight loss in drying is around 100%.

The 5'-nor-anhydrovinblastine ditartrate is performed with a Waters HPLC with diode array detector model 996 (PDA) and pump model 515. A C-18 250 mm x 4 mm L1 (5 µ) column is used and a detection is performed at 269 nm. The mobile phase is comprised of a buffer solution with pH 7.25 (sodium chloride, ammonium acetate, cetyltrimethylammonium bromide) and acetonitrile in a ratio of 60:40.

It is worth mentioning that no references to procedures for the production of 5' -nor-anhydrovinblastine ditartrate with crude 5' -nor-anhydrovinblastine were found in the present state of the art. Moreover, the two proposed alternatives attain a maximum yield without loss of active matter during preparation.

### EXAMPLE

40 kg of fresh Catharanthus roseus leaves and shoots ground in a hammer mill are extracted at room temperature (20° C to 30° C) three times with methanol, with 60 litres for 24 hours each time. The extracts are concentrated at reduced pressure, at a temperature below 30° C, with a volume of up to 20 litres. The recovered methanol is rectified to separate it from the water and is reused in the process. 20 litres of sulphuric acid 1 N are added to the concentrate -mainly comprised of water from the same plant-, and are extracted with methylene chloride three times with 6 litres each time to eliminate chlorophyll and other pigments. The methylene chloride is evaporated, and recovered for reuse by condensation. The aqueous phase is alkalinised with ammonium hydroxide concentrated up to pH 8.5, saturated with sodium chloride, extracted with methylene chloride three times with 6 litres each time and the extracts are concentrated at reduced pressure until dry. The methylene chloride is recovered for reuse by condensation. 75 grams of a crude of total alkaloids are obtained and chromatographed in a column loaded with 500 g of silicagel 60 from 63 to 37 microns (mm) (230-400 mesh ASTM), eluting with methylene chloride-ethyl acetate 80:20. Fractions of 600 ml are obtained. Fractions 3-5 contain the mixture of Vindoline and Catharanthine (fractions are chromatographically controlled on a thin layer in Merck silicagel sheets, using a mixture of methylene chloride-acetone-methanol 92:5:3 or ethyl acetate as development solvent). The separated fractions are evaporated at reduced pressure, thus obtaining 13.6 grams of mixture rich in Vindoline and Catharanthine, with a real mass content of 32.5% of Vindoline and 22.5% of Catharanthine measured by HPLC. A rectification is performed and solvents are recovered and reused after analysis.

4.25 g of glycine and 3.33 g of sodium chloride are dissolved in 540 ml of water and 525 ml of HCl 0,1 N in a glass reactor at room temperature and the mixture is nitrogen bubbled. 31.85 g of ferric chloride hexahydrate are added to this buffer at room temperature with agitation and nitrogen bubbling. 21 g of the chromatographic fraction rich in Vindoline and Catharanthine which contains 55% of the mixture Vindoline:Catharanthine in a molar ratio of 1.1: 1 approximately are added while maintaining the agitation and nitrogen bubbling for 20 hours. A mixture of 1.05 grams of sodium borohydride in 125 ml of concentrated ammonium hydroxide is slowly added to the resulting solution at room temperature and it is left to react for 20 minutes. The mixture is extracted with methylene chloride (4 x 400 ml), the extract is filtered with Celite (100 g) and concentrated at reduced pressure. While the solvent is recovered by condensation to be reused in extraction, the residue is diluted in 75 ml of methylene chloride and filtered on activated carbon layer (7 g). The decoloured solution is concentrated at reduced pressure. The evaporation residue is dissolved in 15 ml of methanol at room temperature and maintained at 2 to 4° C for 12 hours. The precipitate is filtered, washed in cold methanol (10 ml) and vacuum dried. 7.35 g of anhydrovinblastine are thus obtained.

10.1 g of anhydrovinblastine are introduced in a glass reactor, equipped with magnetic stirrer, nitrogen inlet and funnel. The reactor is put in a bath at -60° C. Through the funnel, a solution containing 2.5 g of N-bromosuccinimide, 40 ml methylene chloride and 1.39 ml of trifluoroacetic acid is added with nitrogen bubbling. The mixture is agitated for 2 hours at - 60° C with nitrogen bubbling. After that, the reactor is removed from the bath, the nitrogen bubbling is stopped, a solution of 2.61 g of ammonium acetate in 10.1 ml of water is added and a solution of 2.72 g of silver tetrafluoroborate in 15.7 ml of tetrahydrofurane and 15.7 ml of water is added in turn. After 18 hours at 20° C, 80 ml of sodium bicarbonate 10 % are added, the organic phase is separated and the aqueous phase is extracted again with methylene chloride (3 x 50 ml). The obtained extracts are dried on anhydrous sodium sulphate and filtered, and the solvent is evaporated at reduced pressure, condensed and reused.

The crude product of the reaction is purified by column chromatography, using 500 grams of silicagel 60 from 63 to 37 microns (230-400 mesh ASTM) in a column of 10 cm diameter and eluting with acetone gradients until acetone-methanol in azeotropic relationship is reached, which allows recovery of the mixture simply by condensation after the 5' -nor-anhydrovinblastine concentration.

The fractions' composition is chromatographically controlled in thin layer (Merck silicagel sheets, development solvent: methylene chloride-hexane-methanol 50:40:10). The fractions which contain 5' -nor-anhydrovinblastine as their major component -chromatographically controlled in thin layer- are concentrated at reduced pressure and the residue is rechromatographied. All fractions containing 5' -nor-anhydrovinblastine as only stain are concentrated at reduced pressure and the residue is recrystallised of methanol, dissolving it at room temperature (25° C to 30° C) and letting it crystallise at -20° C, it is then filtered and dried. The material purity is determined by HPLC, being over 99%. 3.15 g of crude 5'-nor-anhydrovinblastine are thus obtained.

In a glass reactor, 2.83 g of crude 5' -nor-anhydrovinblastine with a purity of over 99% (HPLC) are introduced in 14 ml of absolute ethanol and 1.089 g of tartaric acid (equimolar quantity) are added at room temperature.

The mixture is concentrated until dry at reduced pressure: The amorphous white solid is vacuum dried at 666 Pa(5 mmHg), at 40° C in the dark, until constant weight is reached. 5' -nor-anhydrovinblastine ditartrate is quantitatively obtained: 3.92 g. The material purity is determined by HPLC, being over 99%, and its titer determined by titration of total alkaloids by non-aqueous volumetry and corrected for weight loss in drying is around 100%.

The global molar yield of the synthesis from Catharanthine reaches 20% and an approximate mass yield of 50 ppm of 5'-nor-anhydrovinblastine ditartrate with a purity over or equal to 99% in relation to the fresh Catharanthus roseus.

## Claims

1. A procedure for the production of 5' -nor-anhydrovinblastine ditartrate from plants of genus Catharanthus as starting material comprising the following process steps:
• grinding of plants of Apocinaceous family, by means of a blade or hammer mill;
• solid-liquid liberation of alkaloids from the ground plant by means of an organic solvent soluble in water;
• concentration of the extract at room temperature (from 20°C to 30°C) to eliminate the organic solvent and avoid decomposition of Vindoline and Catharanthine;
• recovery of the solvent by rectification;
• dilution of the extract in an aqueous acidic solution;
• elimination of impurities and pigments of the plant extract by means of liquid-liquid extraction of the aqueous acidic dilution, with an organic solvent;
• recovery of solvent by distillation;
• neutralisation and/or alkalisation of the aqueous acidic phase with either an inorganic or organic base;
• liquid-liquid extraction with an organic solvent, which extracts the alkaloids dissolved in water;
• concentration by evaporation, at a temperature below 30°C, of the total alkaloids extract;
• recovery of solvents by condensation;
• separation by liquid chromatography of the total alkaloids extract on silicagel with mixtures of organic solvents;
• isolation of the impure chromatographic fraction rich in Vindoline and Catharanthine;
• concentration until dry by evaporation at reduced pressure;
• recovery of solvents by condensation;
• synthesis of anhydrovinblastine from Vindoline and Catharanthine by dissolving them in a tamponed aqueous acidic solution and adding a ferric salt, which catalyses the binding of Vindoline to Catharanthine resulting in an oxidised iminio intermediate compound;
• tandem reduction of the oxidised iminio intermediate to anhydrovinblastine, with sodium borohydride in an ammonium hydroxide solution;
• extraction of anhydrovinblastine with an organic solvent;
• concentration by evaporation of the organic solvent at low temperature;
• recovery of solvents by condensation;
• crystallisation of anhydrovinblastine from methanol;
• filtering and vacuum drying of anhydrovinblastine;
• reaction of anhydrovinblastine and a halogenated agent at low temperature from -40° C to -80° C with an organic solvent, organic acid and nitrogen bubbling, attaining a halogenated intermediate of anhydrovinblastine;
• reaction of this halogenated intermediate with silver tetrafluoroborate in a medium alkalinised with ammonium acetate in water and tetrahydrofurane to attain crude 5' -nor-anhydrovinblastine;
• extraction with non-polar organic solvent and concentration by evaporation;
• recovery of solvents by condensation;
• purification of 5' -nor-anhydrovinblastine by chromatography and crystallisation until a concentration over 99 % is reached;
• recovery of the chromatographic eluent solvents by condensation of the evaporated fractions;
• formation of 5' -nor-anhydrovinblastine ditartrate
**characterised in that**:
said plants of Apocinaceous family are ground fresh and moist or dried at a temperature less than 30°C,
said synthesis of anhydrovinblastine is performed with an impure fraction of a mixture rich in Vindoline and Catharanthine at pH 2 for a period of between 2 to 40 hours and no Vinblastine is produced,
said anhydrovinblastine is stored after its synthesis in nitrogen and at less than -20°C until its use,
said reaction of said halogenated intemediate with silver tetrafluoroborate is a tandem reaction,
said formation of 5' -nor-anhydrovinblastine ditartrate consists of dissolution of crude 5' -nor-anhydrovinblastine, with a purity of over 99% in absolute ethanol in a concentration of 0,10 to 0,20 mmol/ml and addition of the equimolar quantity of tartaric acid and concentration until dry at reduced pressure and in the dark, or lyophilization.

2. The process of claim 1 wherein the production of 5' -nor-anhydrovinblastine ditartrate is **characterised by** the use of fresh and moist plants, particularly ground leaves and shoots.

3. The process of claim 1 wherein the production of 5' -nor-anhydrovinblastine ditartrate is **characterised by** the use of plants dried at a temperature below 20° C for a period of less than 15 days and ground.

4. The process of claim 1 wherein the production of 5' -nor-anhydrovinblastine ditartrate is **characterised by** the separation by liquid chromatography of the total alkaloids extract on silicagel with mixtures of organic solvents like methylene chloride and ethyl acetate.

5. The process of claim 1 wherein the production of 5' -nor-anhydrovinblastine ditartrate is **characterised by** the anhydrovinblastine synthesis through ferric ion catalyser from Vindoline and Catharanthine together as impure fraction, as resulting from the purification by flash chromatography of the total alkaloids extract, without having been separated from each other and in a concentration range from 40 to 80 % of the impure extract of Catharanthine and Vindoline.

6. The process of claim 1 wherein the production of 5' -nor-anhydrovinblastine ditartrate is **characterised by** the recuperation for reuse of solvents in the different stages of the process.

7. The process of claim 1 wherein the production of 5' -nor-anhydrovinblastine ditartrate is **characterised by** the reaction of anhydrovinblastine and an halogenated agent with N-bromosuccinamide at low temperature between - 40° C and - 80° C with organic solvent, organic acid and nitrogen bubbling, thus obtaining a halogenated intermediate bromo -7 indolenine of anhydrovinblastine.

8. The process of claim 1 wherein the production of 5' -nor-anhydrovinblastine ditartrate is **characterised by** the reaction of the intermediate of 7'-bromoindolenine of anhydrovinblastine to obtain 5'-nor -anhydrovinblastine performed in tandem with bromation, by addition to the same reaction medium -after neutralisation with ammonium acetate- of silver tetrafluoroborate in aqueous medium or mixtures of water and an organic solvent such as tetrahydrofurane or ethyl acetate, without isolation of the intermediate.

9. The process of claim 1 wherein the production of 5' -nor-anhydrovinblastine ditartrate is **characterised by** the flash column liquid chromatographic purification of crude 5-nor-anhydrovinblastine with silicagel and elution with mixtures of acetone-methanol and crystallisation of methanol at a temperature between 2° C and -20° C.

10. The process of claim 1 wherein the production of 5' -nor-anhydrovinblastine ditartrate is **characterised by** the formation of 5'-nor-anhydrovinblastine ditartrate from crude 5' -nor-anhydrovinblastine and the tartaric acid made in absolute alcohol medium, concentrated by evaporation at reduced pressure and dried in the dark.

11. The process of claim 1 wherein the production of 5' -nor-anhydrovinblastine ditartrate is **characterised by** the formation of 5'-nor-anhydrovinblastine ditartrate from crude 5' -nor-anhydrovinblastine and tartaric acid made in aqueous medium and lyophilised.

12. The process of claim 1 wherein the production of 5' -nor-anhydrovinblastine ditartrate is **characterised by** the storage of anhydrovinblastine in an inert nitrogen or argon atmosphere and at -10°C to -80° C, especially at -20° C for more than three months, without evidence of degradation in thin-layer chromatography.

## Patentansprüche

1. Verfahren zur Herstellung von 5'-Nor-Anhydrovinblastin-ditartrat aus Pflanzen der Gattung Catharanthus als Ausgangsmaterial umfassend die folgenden Verfahrensschritte:
• Zerreiben von Pflanzen der Apocinaceousfamilie mittels einer Blatt- oder Hammermühle;
• Fest-Flüssig-Freisetzung von Alkaloiden aus der zerriebenen Pflanze mittels eines organischen, in Wasser löslichen Lösungsmittels;
• Konzentrierung des Extrakts bei Raumtemperatur (von 20°C bis 30°C), um das organische Lösungsmittel zu entfernen und die Zersetzung von Vindolin und Catharanthin zu vermeiden;
• Wiedergewinnung des Lösungsmittels durch Rektifikation;
• Verdünnen des Extrakts in einer wässrigen, säurehaltigen Lösung;
• Entfernung von Verunreinigungen und Pigmenten des Pflanzenextrakts mittels Flüssig-Flüssig-Extraktion der wässrigen, säurehaltigen, verdünnten Lösung mit einem organischen Lösungsmittel;
• Wiedergewinnung von Lösungsmittel durch Destillation;
• Neutralisation und/oder Alkalisierung der wässrigen, säurehaltigen Phase entweder mit einer anorganischen oder organischen Base;
• Flüssig-Flüssig-Extraktion mit einem organischen Lösungsmittel, welches die in Wasser gelösten Alkaloide extrahiert;
• Konzentrierung durch Abdampfen bei einer Temperatur unter 30°C des gesamten Alkaloid-Extrakts;
• Wiedergewinnung der Lösungsmittel durch Kondensation;
• Abtrennung des gesamten Alkaloid-Extrakts durch Flüssigkeitschromatographie auf Silicagel mit Mischungen organischer Lösungsmittel;
• Isolierung der unreinen Chromatographiefraktion, welche reich an Vindolin und Catharanthin ist;
• Konzentrierung zur Trockenheit durch Abdampfen bei reduziertem Druck;
• Wiedergewinnung von Lösungsmitteln durch Kondensation;
• Synthese von Anhydrovinblastin aus Vindolin und Catharanthin durch Auflösen derselben in einer tamponierten (tamponed) wässrigen, säurehaltigen Lösung und Zugabe eines Eisensalzes, welches die Bindung von Vindolin an Catharanthin katalysiert, um ein oxidiertes Iminio-Zwischenprodukt zu ergeben;
• Tandemreduktion des oxidierten Iminio-Zwischenprodukts zu Anhydrovinblastin mit Natriumborhydrid in einer Ammoniumhydroxidlösung;
• Extraktion von Anhydrovinblastin mit einem organischen Lösungsmittel;
• Konzentrierung durch Abdampfen des organischen Lösungsmittels bei niedriger Temperatur;
• Wiedergewinnung von Lösungsmitteln durch Kondensation;
• Kristallisation von Anhydrovinblastin aus Methanol;
• Filtrieren und Vakuumtrocknen von Anhydrovinblastin;
• Reaktion von Anhydrovinblastin und einem halogenierten Reagenz bei niedriger Temperatur von -40°C bis -80°C mit einem organischen Lösungsmittel, organischer Säure und unter Stickstoffeinleitung, um ein halogeniertes Intermediat von Anhydrovinblastin zu gewinnen;
• Reaktion dieses halogenierten Intermediats mit Silbertetrafluorborat in einem mit Ammoniumacetat alkalisiertem Medium in Wasser und Tetrahydrofuran, um rohes 5'-Nor-Anhydrovinblastin zu gewinnen;
• Extraktion mit einem nicht-polaren organischen Lösungsmittel und Konzentrierung durch Abdampfen;
• Wiedergewinnung von Lösungsmitteln durch Kondensation;
• Reinigung von 5'-Nor-Anhydrovinblastin durch Chromatographie und Kristallisation, bis eine Konzentration über 99% erreicht ist;
• Wiedergewinnung der chromatographischen Eluent-Lösungsmittel durch Kondensation der abgedampften Fraktionen;
• Bildung von 5'-Nor-Anhydrovinblastin-ditartrat
**dadurch gekennzeichnet, dass**:
die Pflanzen der Apocinaceous-Familie frisch und feucht zerrieben werden oder bei einer Temperatur von weniger als 30°C getrocknet werden,
die Synthese von Anhydrovinblastin mit einer unreinen Fraktion einer Vindolin- und Catharanthin-reichen Mischung bei pH 2 während einem Zeitraum von 2 bis 40 Stunden durchgeführt wird und kein Vinblastin hergestellt wird,
das Anhydrovinblastin nach seiner Synthese bis zu seiner Verwendung in Stickstoff und bei weniger als -20°C gelagert wird,
die Reaktion des halogenierten Zwischenprodukts mit Silbertetrafluorborat eine Tandemreaktion ist;
die Bildung von 5'-Nor-Anhydrovinblastin-ditartrat aus der Auflösung von rohem 5'-Nor-Anhydrovinblastin mit einer Reinheit von über 99% in absolutem Ethanol in einer Konzentration von 0,10 bis 0,20 mMol/ml und Zugabe der äquimolaren Menge an Weinsäure und Konzentrierung bis zu Trockenheit bei reduziertem Druck und in der Dunkelheit oder Gefriertrocknung besteht.

2. Das Verfahren gemäß Anspruch 1, wobei die Herstellung von 5'-Nor-Anhydrovinblastin-ditartrat **gekennzeichnet ist durch** die Verwendung von frischen und feuchten Pflanzen, insbesondere von zerriebenen Blättern und Schösslingen.

3. Das Verfahren gemäß Anspruch 1, wobei die Herstellung von 5'-Nor-Anhydrovinblastin-ditartrat **gekennzeichnet ist durch** die Verwendung von Pflanzen, getrocknet bei einer Temperatur unter 20°C während eines Zeitraums von weniger als 15 Tagen und zerrieben.

4. Das Verfahren gemäß Anspruch 1, wobei die Herstellung von 5'-Nor-Anhydrovinblastin-ditartrat **gekennzeichnet ist durch** die Abtrennung des gesamten Alkaloidextrakts **durch** Flüssigkeitschromatographie auf Silicagel mit einer Mischung von organischen Lösungsmitteln wie Methylenchlorid und Ethylacetat.

5. Das Verfahren gemäß Anspruch 1, wobei die Herstellung von 5'-Nor-Anhydrovinblastin-ditartrat **gekennzeichnet ist durch** die Anhydrovinblastinsynthese mit Eisenion-Unterstützung aus Vindolin und Catharanthin zusammen als unreine Fraktion, wie aus der Reinigung **durch** Flash-Chromatographie des Gesamtalkanoid-extrakts stammend, ohne voneinander getrennt worden zu sein und in einem Konzentrationsbereich von 40 bis 80% des unreinen Extrakts von Catharanthin und Vindolin.

6. Das Verfahren gemäß Anspruch 1, wobei die Herstellung von 5'-Nor-Anhydrovinblastin-ditartrat **gekennzeichnet ist durch** die Zurückgewinnung von Lösungsmitteln zur Wiederverwendung in den verschiedenen Stufen des Verfahrens.

7. Das Verfahren gemäß Anspruch 1, wobei die Herstellung von 5'-Nor-Anhydrovinblastin-ditartrat **gekennzeichnet ist durch** die Reaktion von Anhydrovinblastin und einem halogenierten Reagenz mit N-Bromsuccinamid bei niedriger Temperatur zwischen -40°C und -80°C mit organischem Lösungsmittel, organischer Säure und Stickstoffeinleitung, um somit ein halogeniertes Zwischenprodukt Brom-7-indolenin von Anhydrovinblastenin zu erhalten.

8. Das Verfahren gemäß Anspruch 1, wobei die Herstellung von 5'-Nor-Anhydrovinblastin-ditartrat **gekennzeichnet ist durch** die Reaktion des Zwischenprodukts von 7'-Bromindolenin von Anhydrovinblastin, um 5'-Nor-Anhydrovinblastin zu erhalten, im Tandem mit der Bromierung **durch** Zugabe von Silbertetrafluorborat zum gleichen Reaktionsmedium, nach Neutralisation mit Ammoniumacetat, in wässrigem Medium oder Mischungen von Wasser und einem organischen Lösungsmittel wie z. B. Tetrahydrofuran oder Ethylecetat, ohne Isolierung des Zwischenprodukts.

9. Das Verfahren gemäß Anspruch 1, wobei die Herstellung von 5'-Nor-Anhydrovinblastin-ditartrat **gekennzeichnet ist durch** Flash-Säulen-Flüssigkeitschromatographie-Reinigung von rohem 5'-Nor-Anhydrovinblastin mit Silicagel und Eluierung mit Mischungen von Aceton-Methanol und Kristallisation aus Methanol bei einer Temperatur zwischen 2°C und -20°C.

10. Das Verfahren gemäß Anspruch 1, wobei die Herstellung von 5'-Nor-Anhydrovinblastin-ditartrat **gekennzeichnet ist durch** die Bildung von 5'-Nor-Anhydrovinblastin-ditartrat aus rohem 5'-Nor-Anhydrovinblastin und der Weinsäure, dargestellt in absolutem Alkoholmedium, konzentriert **durch** Abdampfen bei reduziertem Druck, und getrocknet in der Dunkelheit.

11. Das Verfahren gemäß Anspruch 1, wobei die Herstellung von 5'-Nor-Anhydrovinblastin-ditartrat **gekennzeichnet ist durch** die Bildung von 5'-Nor-Anhydrovinblastin-ditartrat aus rohem 5'-Nor-Anhydrovinblastin und Weinsäure, hergestellt in wässrigem Medium und gefriergetrocknet.

12. Das Verfahren gemäß Anspruch 1, wobei die Herstellung von 5'-Nor-Anhydrovinblastin-ditartrat **gekennzeichnet ist durch** die Lagerung von Anhydrovinblastin in einer inerten Stickstoff- oder Argonatmosphäre und bei -10°C bis -80°C, insbesondere bei -20°C für mehr als drei Monate, ohne Hinweis auf Zersetzung bei Dünnschichtchromatographie.

## Revendications

1. Procédure pour la production de ditartrate de 5'-noranhydrovinblastine à partir de plantes du genre Catharanthus en tant que matière de départ comprenant les étapes de traitement suivantes
• broyage de plantes de la famille des Apocinacées, au moyen d'un broyeur à lames ou à marteaux ;
• libération solide-liquide d'alcaloïdes provenant de la plante broyée au moyen d'un solvant organique soluble dans l'eau ;
• concentration de l'extrait à la température ambiante (de 20°C à 30°C) pour éliminer le solvant organique et éviter une décomposition de la Vindoline et de la Catharanthine ;
• récupération du solvant par rectification ;
• dilution de l'extrait dans une solution acide aqueuse ;
• élimination des impuretés et des pigments de l'extrait de plante au moyen d'une extraction liquide-liquide de la dilution acide aqueuse, par un solvant organique;
• récupération du solvant par distillation ;
• neutralisation et/ou alcalinisation de la phase acide aqueuse avec une base soit inorganique, soit organique ;
• extraction liquide-liquide par un solvant organique, qui extrait les alcaloïdes dissous dans l'eau ;
• concentration par évaporation à une faible température inférieure à 30°C de l'extrait total d'alcaloïdes ;
• récupération des solvants par condensation ;
• séparation par chromatographie liquide de l'extrait total d'alcaloïdes sur un gel de silice avec des mélanges de solvants organiques ;
• isolation de la fraction chromatographique impure riche en Vindoline et en Catharanthine ;
• concentration à siccité par évaporation sous pression réduite ;
• récupération des solvants par condensation ;
• synthèse d'anhydrovinblastine à partir de la Vindoline et de la Catharanthine par dissolution de celles-ci dans une solution acide aqueuse tamponnée et addition d'un sel ferrique, qui catalyse la liaison de la Vindoline à la Catharanthine et conduit à un composé intermédiaire imino oxydé ;
• réduction en tandem de l'intermédiaire imino oxydé en anhydrovinblastine, avec du borohydrure de sodium dans une solution d'hydroxyde d'ammonium ;
• extraction de l'anhydrovinblastine par un solvant organique ;
• concentration par évaporation du solvant organique à basse température ;
• récupération des solvants par condensation ;
• cristallisation de l'anhydrovinblastine dans du méthanol ;
• filtration et séchage sous vide de l'anhydrovinblastine ;
• réaction de l'anhydrovinblastine et d'un agent halogéné à une basse température de -40°C à -80°C en présence d'un solvant organique, d'un acide organique et d'un barbotage d'azote, pour obtenir un intermédiaire halogéné de 1' anhydrovinblastine ;
• réaction de cet intermédiaire halogéné avec du tétrafluoroborate d'argent dans un milieu alcalinisé par de l'acétate d'ammonium dans l'eau et du tétrahydrofurane pour obtenir la 5'-nor-anhydrovinblastine brute ;
• extraction par un solvant organique non polaire et concentration par évaporation ;
• récupération des solvants par condensation ;
• purification de la 5'-nor-anhydrovinblastine par chromatographie et cristallisation jusqu'à obtention d'une concentration supérieure à 99% ;
• récupération des solvants formant éluant chromatographique par condensation des fractions évaporées ;
• formation du ditartrate de 5'-nor-anhydrovinblastine,
**caractérisée en ce que**
lesdits plantes de la famille des Apocinacées sont broyées alors qu'elles sont fraîches et humides ou séchées à une température inférieure à 30°C,
ladite synthèse de 1'anhydrovinblastine est effectuée avec une fraction impure d'un mélange riche en Vindoline et en Catharanthine à pH 2 pendant une période comprise entre 2 et 40 heures et qu'aucune Vinblastine n'est produite,
ladite anhydrovinblastine est stockée après sa synthèse dans de l'azote et à moins de-20°C jusqu'au moment de son utilisation,
ladite réaction dudit intermédiaire halogéné avec le tétrafluoroborate d'argent est une réaction en tandem,
ladite formation du ditartrate de 5'-nor-anhydrovinblastine comprend la dissolution de la 5'-nor-anhydrovinblastine brute, avec une pureté supérieure à 99% dans de l'éthanol absolu à une concentration de 0,10 à 0,20 mmole/ml et l'addition d'une quantité équimolaire d'acide tartrique et la concentration à siccité Bous pression réduite et dans l'obscurité, ou une lyophilisation.

2. Procédé selon la revendication 1, dans lequel la production du ditartrate de 5'-nor-anhydrovinblastine est **caractérisée par** l'utilisation de plantes fraîches et humides, en particulier de feuilles et de pousses broyées.

3. Procédé selon la revendication 1, dans lequel la production du ditartrate de 5'-nor-anhydrovinblastine est **caractérisée par** l'utilisation de plantes séchées à une température inférieure à 20°C pendant une période inférieure à 15 jours, puis broyées.

4. Procédé selon la revendication 1, dans lequel la production du ditartate de 5'-nor-anhydrovinblastine est **caractérisée par** la séparation par chromatographie liquide de l'extrait total d'alcaloïdes sur un gel de silice avec des mélanges de solvants organiques comme le chlorure de méthylène et l'acétate d'éthyle.

5. Procédé selon la revendication 1, daos lequel la production du ditartrate de 5'-nor-anhydrovinblastine est **caractérisée par** la synthèse de l'anhydrovinblastine par l'intermédiaire d'un catalyseur comportant un ion ferrique à partir de Vindoline et de Catharanthine ensemble en tant que fraction impure, telle qu'elle provient de la purification par chromatographie éclair de l'extrait total d'alcaloïdes, sans avoir été séparée l'une de l'autre et dans une gamme de concentration de 40 à 80% de Catharanthine et de Vindoline dans l'extrait impur.

6. Procédé selon la revendication 1, dans lequel la production du ditartrate de 5'-nor-anhydrovinblastine est **caractérisée par** la récupération pour réutilisation des solvants dans les différentes étapes du procédé.

7. Procédé selon la revendication 1, dans lequel la production du ditartrate de 5'-nor-anhydrovinblastine est **caractérisée par** la réaction de l'anhydrovinblastine et d'un agent halogéné avec du N-bromosuccinamide à une température basse comprise entre -40°C et -80°C en présence d'un solvant organique, d'un acide organique et d'un barbotage d'azote, de façon à obtenir un intermédiaire halogéné bromo-7-indolénine d'anhydrovinblastine.

8. Procédé selon la revendication 1, dans lequel la production du ditartrate de 5'-nor-anhydrovinblastine est **caractérisée par** la réaction de l'intermédiaire 7'-bromoindolénine d'anhydrovinblastine pour obtenir la 5'-nor-anhydrovinblastine, réalisée en tandem avec une bromation, par addition au même milieu de réaction, après neutralisation avec de l'acétate d'ammonium, de tétrafluoroborate d'argent dans un milieu aqueux ou dans des mélanges d'eau et d'un solvant organique comme le tétrahydrofurane ou l'acétate d'éthyle, sans isolation de l'intermédiaire.

9. Procédé selon la revendication 1, dans lequel la production du ditartrate de 5'-nor-anhydrovinblastine est **caractérisée par** la purification chromatographique liquide sur colonne éclair de la 5'-nor-anhydrovinblastine et gel de silice et une élution avec des mélanges d'acétone et de méthanol et la cristallisation dans le méthanol à une température comprise entre 2°C et - 20°C.

10. Procédé selon la revendication 1, dans lequel la production du ditartrate de 5'-nor-anhydrovinblastine est **caractérisée par** la formation du ditartrate de 5'-nor-anhydrovinblastine à partir de 5'-nor-anhydrovinblastine brute et d'acide tartrique réalisée dans un milieu d'alcool absolu, la concentration par évaporation sous pression réduite et le séchage à l'obscurité.

11. Procédé selon la revendication 1, dans lequel la production du ditartrate de 5'-nor-anhydrovinblastine est **caractérisée par** la formation de ditartrate de 5'-nor-anhydrovinblastine à partir de 5'-nor-anhydrovinblastine brute et d'acide tartrique effectuée dans un milieu aqueux, puis lyophilisation.

12. Procédé selon la revendication 1, dans lequel la production du ditartrate de 5'-nor-anhydrovinblastine est **caractérisée par** le stockage de l'anhydrovinblastine dans une atmosphère inerte d'azote ou d'argon, à une température de -10°C à -80°C, en particulier à -20°C pendant plus de trois mois, sans observation d'une dégradation par chromatographie en couche mince.
